(19) Europäisches Patentamt
European Patent Office
Office européen des brevets

(11) **EP 4 455 305 A1**

(12) **EUROPEAN PATENT APPLICATION**
published in accordance with Art. 153(4) EPC

(43) Date of publication:
**30.10.2024 Bulletin 2024/44**

(21) Application number: **22909929.6**

(22) Date of filing: **19.12.2022**

(51) International Patent Classification (IPC):
***C12Q 1/6851*** (2018.01)   ***C12N 15/11*** (2006.01)

(86) International application number:
**PCT/CN2022/139965**

(87) International publication number:
**WO 2023/116616 (29.06.2023 Gazette 2023/26)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **22.12.2021 CN 202111581332**

(71) Applicant: **Beijing Zephyrm Biotechnology Co., Ltd.**
**Beijing 102200 (CN)**

(72) Inventors:
• **ZHANG, Yu**
  **Beijing 102200 (CN)**

• **GAN, Yidi**
  **Beijing 102200 (CN)**
• **ZHANG, Shuai**
  **Beijing 102200 (CN)**
• **LUO, Yanchao**
  **Beijing 102200 (CN)**
• **XU, Ranran**
  **Beijing 102200 (CN)**
• **WANG, Shuyan**
  **Beijing 102200 (CN)**

(74) Representative: **E. Blum & Co. AG**
**Franklinturm**
**Hofwiesenstrasse 349**
**8050 Zürich (CH)**

(54) **METHOD FOR TESTING EXPRESSION LEVEL OF PLURIPOTENCY GENE**

(57)    The present invention relates to the use of a reference cell for testing pluripotency gene expression level, wherein the reference cell is selected from cells with low and stable expression level of a pluripotency gene. Preferably, the reference cell is selected from HFF cells, optionally, the pluripotency gene is selected from OCT4, NANOG. The present invention also relates to primers and probes for OCT4 and NANOG. The present invention also relates to a method for testing pluripotency gene expression level. The present invention further relates to a use of the detection method in detecting a hPSC residue level and characterizing a hPSC differentiation process. The method of the present invention has the advantages of simplicity, stability, high sensitivity, wide application range, and high accessibility. Therefore, it is easy to standardize and can provide a unified standard for testing cell pluripotency gene expression level.

Figure 4

## Description

The present application claims the priority of the Chinese patent application with application number 202111581332.X.

## Technical Field

The present invention relates to the biological field, specifically to a method for testing the expression levels of pluripotency genes and related uses thereof.

## Background Art

Currently, several methods exist for testing expression levels of pluripotency genes, including immune hybridization technology, RT-PCR technology and fluorescence quantitative PCR technology, etc. Among them, the fluorescence quantitative PCR technology is widely accepted in the industry due to its advantages of specificity and sensitivity. However, it has limitations when detecting pluripotency gene expression levels, such as the absence universal reference product.CN110573607A discloses an assay for pluripotent stem cells, detailing a method for testing pluripotent stem cells (PSC) residue levels using a reference culture containing PSC as a reference substance. This method is challenging to prepare and is limited to detecting undifferentiated PSC residues, making it unsuitable for general testing of pluripotency gene expression levels in cells.CN110268056A pertains to a method for testing expression levels of pluripotency gene, specifically utilizing human embryonic stem cells (hESCs) as control cells within the context of encapsulted liver tissue research. However, since hESCs are difficult to obtain and not easy to commercialize, and the expression level of the pluripotency gene of hESCs varies greatly under different culture conditions, hESCs are not suitable as a reference cell. Therefore, this method cannot be used as a unified standard method for testing cell pluripotency gene expression level.

Monterubbianesi et al. ("A comparative in vitro study of the osteogenic and adipogenic potential of human dental pulp stem cells, gingival fibroblasts and foreskin fibroblasts", Sci Rep 9, 1761(2019) 9(1):1761) published an in vitro comparative study on the osteogenic and adipogenic potential of human dental pulp stem cells, gingival fibroblasts and foreskin fibroblasts. They specifically mentioned that human foreskin fibroblasts (HFF) cells are very easy to obtain and HFF cells exhibit low expression levels of OCT4 and NANOG genes. Therefore, HFF cells can be utilized for detection of OCT4 and NANOG genes. This indicates that HFF cells have the possess the potential to serve as ideal reference cells for testing pluripotency gene expression levels. Therefore, there remains an unmet need in this field for a simple, stable, and readily standardizable method for testing pluripotency gene expression levels.

## Contents of the present invention

One of the purposes of the present invention is to provide a simple, stable and readily standardizable method for testing pluripotency gene expression levels.

The present invention provides a method for testing the expression levels of cell pluripotency genes OCT4 and NANOG, utilizing reference cells. This method offers several advantages including simplicity, stability, high sensitivity, a broad application range and high accessibility. Therefore, it is easily standardized and can provide a unified standard for evaluating cell pluripotency gene expression levels. Consequently, the method provided by the present invention can standardize the messy status quo currently existing in this field.

The present invention can be applied for evaluating the pluripotency of human pluripotent stem cells (hPSCs), testing the residue levels of hPSCs, characterizing the differentiation process of hPSCs, and assessing the pluripotency gene expression levels in mesenchymal stem cells, among other related applications

Therefore, in one aspect, the present invention provides a use of a reference cell for testing the expression level of a pluripotency gene. The reference cell is selected from cells with low and stable expression level of the pluripotency gene. Optionally, the reference cell is selected from the group consisting of human foreskin fibroblast (HFF), human skin fibroblast (HSF), bone marrow mesenchymal stem cell (BMMSC), adipose mesenchymal stem cell (ADMSC), umbilical cord mesenchymal stem cell (UCMSC) as well as human primary preadipocyte, human cerebral vascular pericyte, human chondrocyte, human primary aortic smooth muscle cell, and human primary osteoblast. Preferably, HFF cells are selected as the reference cells and the pluripotency gene is selected from OCT4 and NANOG. In one embodiment, testing pluripotency gene expression level comprises selecting an internal control. Optionally, the internal control is selected from either an internal positive control or an internal negative control. Preferably, the internal positive control is the GAPDH gene.

In another embodiment, the reference cell can be used to detect hPSCs residue level in an hPSC-related preparation, or to characterize hPSCs differentiation process.

In yet another embodiment, the pluripotency gene expression level is quantified in the form of $2^{\Delta\Delta Cq}$.

In another aspect, the present invention provides an OCT4 gene detection agent, which comprises an OCT4 gene forward primer sequence, an OCT4 gene reverse primer sequence and optionally an OCT4 gene probe sequence. Optionally, the OCT4 gene forward primer sequence (5'-3') is AGGAAGCTGACAACAATGAA, the OCT4 gene reverse primer sequence (5'-3') is TTGCCTCTCACTCGGTTC, and the OCT4 gene probe sequence (5'-3') is F AM-TTCGCTTTCTCTTTCGGGCCTGC ACG-BHQ1.

In yet another aspect, the present invention provides a NANOG gene detection agent, which comprises a NANOG gene forward primer sequence, a NANOG gene reverse primer sequence, and optionally a NANOG gene probe sequence. Optionally, the NANOG gene forward primer sequence (5'-3') is AACTCTCCAACATCCTGAACCT, the NANOG gene reverse primer sequence (5'-3') is CTGCGTCACACCATTGCTATT, and the NANOG gene probe sequence (5'-3') is FAM-CGGCCAGTTGTTTTTCTGCCACCTCT-BHQ1.

In another aspect, the present invention provides a GAPDH gene detection agent, which comprises a GAPDH gene forward primer sequence, a GAPDH gene reverse primer sequence, and optionally a GAPDH gene probe sequence. Optionally, the GAPDH gene forward primer sequence (5'-3') is GTCTCCTCTGACTTCAACAGCG, the GAPDH gene reverse primer sequence (5'-3') is ACCACCCTGTTGCTGTAGCCAA, and the GAPDH gene probe sequence (5'-3') is FAM-CCTCCACCTTTGACGCTGGGGCTGGCA-BHQ1.

In yet another aspect, the present invention provides a method for testing pluripotency gene expression level, which comprises:

(a) providing a sample to be tested;

(b) providing a reference cell, wherein the reference cell is selected from cells with low and stable expression level of the pluripotency gene. Optionally, the reference cell is selected from the group consisting of human foreskin fibroblast (HFF), human skin fibroblast (HSF), bone marrow mesenchymal stem cell (BMMSC), adipose mesenchymal stem cell (ADMSC), umbilical cord mesenchymal stem cell (UCMSC), human primary preadipocyte, human cerebral vascular pericyte, human chondrocyte, human primary aortic smooth muscle cell, human primary osteoblast. Preferably, the reference cell is HFF cells;

(c) extracting an RNA from the sample to be detected;

(d) testing the expression level of the pluripotency gene, in which OCT4 or NANOG is used as a test gene, and GAPDH is used as an internal reference gene;

(e) determining the pluripotency gene expression level of the sample to be tested by comparing the expression level of the test gene in the sample to be tested with the expression level of the test gene in the reference cells.

In one embodiment, the RNA extraction process comprises two genome removal steps to ensure genome removal efficiency.

In another embodiment, the expression level of the pluripotency gene in the test sample is tested by RT-qPCR, optionally, the non-reverse transcriptase control (NRC) detection result of all genes in RT-qPCR is negative.

In another aspect, the present invention provides a use of the detection method of the present invention in testing hPSC residue level and characterizing hPSC differentiation process.

Beneficial effects of the present invention:

Compared with CN110573607A, the present invention has at least the following differences:

1) The present invention sets up fixed reference cells, and thus can report the pluripotency gene expression level relative to the reference cells, thereby providing a unified standard for the testing and detection of pluripotency gene expression level;

2) In the process of RNA extraction, the present invention strengthens the genome removal process to ensure that the extracted RNA has no genome residues, thereby improving the accuracy of qPCR quantitative results;

3) The primer probe sequences of the OCT4, NANOG and GAPDH genes of the present invention are different from the primer probe sequences of CN110573607A.

In the present invention, the HFF cells sourced from ATCC are used as reference cells, and RT-qPCR method is employed to quantitatively assess the expression levels of OCT4 and NANOG genes in human cells. The method is simple and fast, and the results are stable and reliable. Thus, the technical method of the present invention can be readily implemented in any laboratory and is straightforward to standardize.

**Brief Description of the Drawings**

Figure 1 shows the diagram of dissolution peak results of plasmid amplified by three pairs of OCT4 candidate primers.

Figure 2 shows the diagram of dissolution peak results of plasmid amplified by three pairs of NANOG candidate primers.

Figure 3 shows the diagram of dissolution peak results of plasmid amplified by three pairs of GAPDH candidate primers.

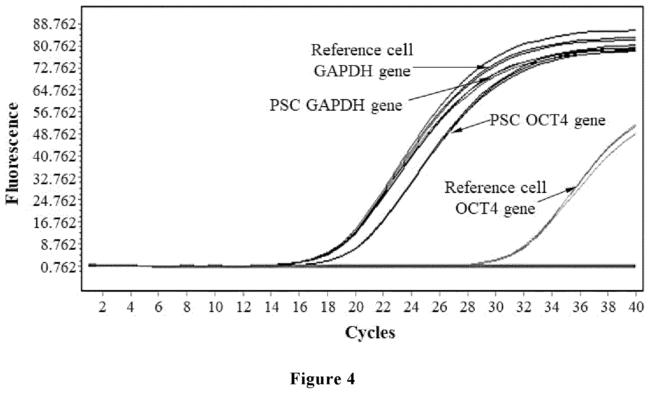

Figure 4 shows the diagram of amplification curve results of testing the expression level of OCT4 gene in pluripotent stem cells (PSC), in which the internal positive control is the GAPDH gene.

**Specific Models for Carrying Out the present invention**

The present invention can be implemented through the following examples, but the present invention is not limited thereto.

In the present invention, an RT-qPCR detection method for pluripotency genes OCT4 and NANOG using HFF cells as reference cells was established. The HFF cells were used as the "ruler" of gene expression level to describe the pluripotency gene expression level and cell pluripotency level of the cells to be tested.

Experimental Materials:

Reference cells HFF and other cells were purchased from ATCC.

The primers and probes of OCT4, NANOG, and GAPDH genes were synthesized by Genscript Biotechnology Co., Ltd.

Experimental reagents:

DMEM modified medium was purchased from ATCC.

FBS was purchased from Thermo Fisher Scientific.

Penicillin-Stretomycin was purchased from Thermo Fisher Scientific.

FastPure® Cell/Tissue Total RNA Isolation Kit was purchased from Nanjing Vazyme Biotech Co., Ltd.

HiScript® II U+ One Step qRT-PCR Probe Kit was purchased from Nanjing Vazyme Biotech Co., Ltd.

HiScript® II One Step qRT-PCR SYBR® Green Kit was purchased from Nanjing Vazyme Biotech Co., Ltd.

ChamQ Geno-SNP Probe Master Kit was purchased from Nanjing Vazyme Biotech Co., Ltd.

$\beta$-Mercaptoethanol was purchased from Thermo Fisher Scientific.

Anhydrous ethanol was purchased from Shanghai Aladdin Biochemical Technology Co., Ltd.

DNase I was purchased from Nanjing Vazyme Biotech Co., Ltd.

DPBS was purchased from Thermo Fisher Scientific.

RNase-free water was purchased from Nanjing Vazyme Biotech Co., Ltd.

Examples

Example 1: Construction method for reference cell library

The HFF cells used in this example were purchased from ATCC. The newly purchased HFF cells were used as the P0 generation and were continuously passaged using DMEM modified complete medium (containing DMEM modified medium, FBS, and Penicillin-Streptomycin). The P2 generation was used as the main cell bank and the P5 generation was used as the working cell bank.

Example 2: Detection method

2.1 RNA extraction:

FastPure® Cell/Tissue Total RNA Isolation Kit was used to extract RNA from cells to be tested and reference cells.

2.1.1 Reagent preparation:

① Buffer RL1 stock solution in the kit was taken, added with 1% β-mercaptoethanol, and mixed well to prepare Buffer RL1 working solution.

② Buffer RL2 stock solution in the kit was taken, added with twice volume of absolute ethanol, and mixed well to prepare Buffer RL2 working solution.

③ Buffer RW2 stock solution in the kit was taken, added with 2.5 times volume of absolute ethanol, and mixed well to prepare Buffer RW2 working solution.

④ 5 µl of DNase I solution was added to 65 µl of RDD Buffer in the kit, and mixed well to prepare DNase I working solution.

2.1.2 Cell washing

$1 \times 10^6$ to $2.5 \times 10^6$ cells were taken, added to at least 10 times volume of DPBS, and centrifuged at $500 \times g$ for 5 minutes to remove the supernatant. 1 ml of DPBS was added to resuspend the cells, centrifuged at $500 \times g$ for 5 minutes to remove the supernatant, and this operation was repeated twice.

2.1.3 Lysis

500 µl of the Buffer RL1 working solution was added to the washed cells, and pipetted repeatedly until the cells were completely lysed.

2.1.4 Filtration

The cell lysate was transferred to gDNA-Filter Columns and centrifuged at 12,000 rpm for 2 minutes. The gDNA-Filter Columns was discarded, and the filtrate was retained in the collection tube.

2.1.5 Adsorption

800 µl of the Buffer RL2 working solution was added to the filtrate in the collection tube, pipetted and mixed repeatedly. 650 µl of the mixed solution was pipetted, transferred to RNAPure Columns, and centrifuged at 12,000 rpm for 1 min, and the filtrate was discarded. The remaining mixture was centrifuged using the same procedure.

2.1.6 Removal of protein

500 µl of Buffer RW1 was added to RNAPure Columns, and centrifuged at 12,000 rpm for 1 min, the waste liquid in the collection tube was discarded.

2.1.7 Desalination

700 µl of the Buffer RW2 working solution was added to RNAPure Columns, and centrifuged at 12,000 rpm for 1 min, the waste liquid in the collection tube was discarded.

2.1.8 Removal of genomic DNA

① 70 μl of the DNase I working solution was taken and added dropwise to the center of RNAPure Columns to completely cover the adsorption column membrane, and allowed to stand at room temperature for 15 to 30 minutes.

② 500 μl of Buffer RW1 was added to RNAPure Columns, and centrifuged at 12,000 rpm for 1 minute, the waste liquid in the collection tube was discarded.

③ 700 μl of the Buffer RW2 working solution was added to RNAPure Columns, and centrifuged at 12,000 rpm for 1 minute, the waste liquid in the collection tube was discarded.

2.1.9 The steps for removing genomic DNA were repeated.

2.1.10 Desalination

700 μl of the Buffer RW2 working solution was added to RNAPure Columns, and centrifuged at 12,000 rpm for 1 minute, the waste liquid in the collection tube was discarded.

2.1.11 Removal of residuals

The RNAPure Columns adsorption column was transferred into a new RNase-free centrifuge tube and centrifuged at 12,000 rpm for 2 minutes to remove residual liquid.

2.1.12 Elution

The adsorption column was transferred to a new RNase-free centrifuge tube, 100 μl of RNase-free water was added dropwise to the center of the adsorption column, allowed to stand at room temperature for 2 minutes, and centrifuged at 12,000 rpm for 2 minutes to collect the extracted total RNA solution.

2.1.13 RNA quality determination

RNA concentration and purity were detected with NanoDrop One.

2.2 RT-qPCR amplification:

HiScript® II U+ One Step qRT-PCR Probe Kit and ChamQ Geno-SNP Probe Master Kit were used to test RNA samples for gene expression level and genomic residues.

2.2.1 Preparation of RNA sample with working concentration

The cell RNA to be tested and the reference cell RNA were diluted with sample diluent to 12.5ng/μl to prepare RNA templates with working concentration.

2.2.2 Preparation of RT-qPCR reaction system

The detection (TEST) reaction system and the no-reverse transcriptase control (NRC) reaction system were prepared for each RNA sample, respectively, and 3 duplicate wells were set for each reaction.

① Preparation of TEST reaction system

| Component name | Amount of component added (μl) |
|---|---|
| RNase-free water | 3 |
| 2× One Step U+ Mix | 10 |
| One Step U+ Enzyme Mix | 1 |
| Upstream primer (10μM) | 0.8 |

(continued)

| Component name | Amount of component added (μl) |
|---|---|
| Downstream primer (10μM) | 0.8 |
| Probe (10μM) | 0.4 |
| RNA template or NTC template | 4 |
| Total | 20 |

(2) Preparation of NRC reaction system

NRC reaction system was prepared for the internal control of genomic contamination. The preparation method was as follows:

| Component name | Amount of component added (μl) |
|---|---|
| RNase-free water | 4 |
| 2× ChamQ Geno-SNP Probe Master Mix | 10 |
| Upstream primer (10μM) | 0.8 |
| Downstream primer (10μM) | 0.8 |
| Probe (10μM) | 0.4 |
| RNA template | 4 |
| Total | 20 |

2.2.3 RT-qPCR amplification

After the reaction system was prepared, amplification was performed on a LightCycler 480 II fluorescence quantitative PCR instrument according to the following reaction procedure.

| Reaction program | Number of cycles | Temperature (°C) | Reaction time |
|---|---|---|---|
| Reverse transcription | 1 | 55 | 15min |
| Pre-denaturation | 1 | 95 | 30s |
| Amplification | 40 | 95 | 10s |
|  |  | 60 | 30s |
| Cooling | 1 | 40 | 1min |

2.2.4 Data analysis

After the reaction was completed, Abs Quant/Fit Point mode was selected for analysis, noiseband was set to 1.50000, Threshold was set to 2.00000, the Cq of each sample was recorded, and the mean Cq was calculated.

① Conditions for successful experiment

Under normal circumstances, the Cq of each well of NTC should be >35.00 or not detected;
The Cq of each well of NRC should be >35.00 or not detected.

② Result analysis:

According to the purpose of the experiment, the sample $\Delta Cq$, $\Delta\Delta Cq$ and $2^{-\Delta\Delta Cq}$ were calculated according to the following formulas.

a. Analysis of expression level of target gene relative to internal reference gene GAPDH:

$$\Delta Cq = \text{mean } Cq \text{ value of target gene} - \text{mean } Cq \text{ value of GAPDH gene}$$

b. Analysis of expression level of sample to be tested relative to reference cell:

$$\Delta\Delta Cq = \Delta Cq \text{ of sample to be tested} - \text{reference cell } \Delta Cq$$

Expression level of sample to be tested relative to reference cell = $2^{-\Delta\Delta Cq}$

Example 3: Method study

3.1 Screening of primers and probes

3.1.1 Screening of OCT4 gene primers and probes

OCT4 candidate primers were as follows:

| Primer name | Sequence (5'-3') |
| --- | --- |
| OCT4-F-1 | AAGCGAACCAGTATCGAGAACC |
| OCT4-R-1 | AATCCTCTCGTTGTGCATAGTCG |
| OCT4-F-2 | GTGGAGGAAGCTGACAACAA |
| OCT4-R-2 | ATTCTCCAGGTTGCCTCTCA |
| OCT4-F-3 | AGGAAGCTGACAACAATGAA |
| OCT4-R-3 | TTGCCTCTCACTCGGTTC |

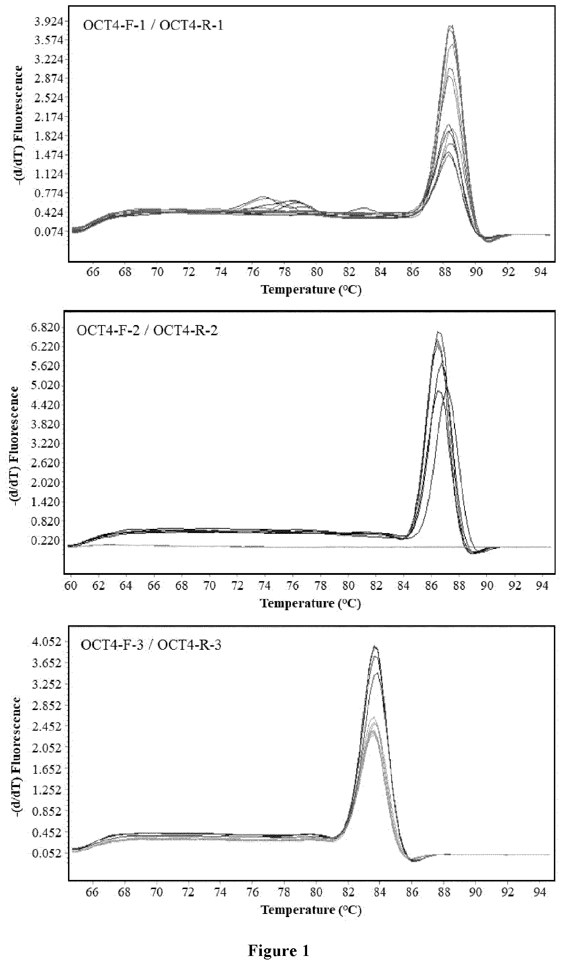

Using pOCT4 plasmid as a template, the three pairs of OCT4 primers were screened for specificity using the dye method qPCR reagent HiScript® II One Step qRT-PCR SYBR® Green Kit (Vazyme, Q221), and the results were shown in Figure 1.
As shown in Figure 1, the amplified products of the three primer pairs showed singlet dissolution peaks, and met the requirements of specificity. However, the melting curve of OCT4-F-3/OCT4-R-3 was more stable, so OCT4-F-3/OCT4-R-3 was preferred for probe design and screening.
OCT4 candidate probes were as follows:

| Probe name | Sequence (5'-3') | Probe label |
| --- | --- | --- |
| OCT4-P-1 | TGGTTCGCTTTCTCTTTCGGGCCTGCA | FAM-BHQ1 |
| OCT4-P-2 | CTGGTTCGCTTTCTCTTTCGGGCCTGC | FAM-BHQ1 |
| OCT4-P-3 | TTCGCTTTCTCTTTCGGGCCTGCACG | FAM-BHQ1 |

Using pOCT4 plasmid as a template, the three OCT4 probes were tested for amplification efficiency using the probe method qPCR reagent HiScript® II U+ One Step qRT-PCR Probe Kit (Vazyme, Q222-CN). The results were shown in Table 1.

Table 1: OCT4 probe amplification efficiency test results

| Probe name | Amplification efficiency | Standard curve error value |
| --- | --- | --- |
| OCT4-P-1 | 87.4% | 0.0658 |
| OCT4-P-2 | 87.1% | 0.0785 |
| OCT4-P-3 | 91.7% | 0.0787 |

Note: "Standard curve error value" was a LightCycler 480 standard curve parameter, and less than 0.2 was the acceptable range.

As shown in Table 1, OCT4-P-3 had the best amplification efficiency, so the primer-probe combination of OCT4-F-3/OCT4-R-3/OCT4-P-3 was preferred for working concentration optimization.

The working concentration ranges and detection results of OCT4 primers and probes were shown in Table 2.

Table 2: Amplification efficiency of OCT4 primer probe at different working concentrations

| No. | OCT4-F-3 Working concentration (nM) | OCT4-R-3 Working concentration (nM) | OCT4-P-3 Working concentration (nM) | Amplification efficiency | Standard curve error value |
|---|---|---|---|---|---|
| 1 | 250 | 250 | 125 | 97.3% | 0.0651 |
| 2 | 250 | 250 | 200 | 99.0% | 0.0338 |
| 3 | 400 | 400 | 200 | 98.1% | 0.0316 |
| 4 | 400 | 400 | 300 | 92.9% | 0.1200 |
| 5 | 400 | 400 | 400 | 96.4% | 0.0178 |
| 6 | 500 | 500 | 250 | 97.4% | 0.0390 |
| 7 | 500 | 500 | 350 | 97.5% | 0.0720 |
| 8 | 500 | 500 | 500 | 98.5% | 0.0221 |

Note: "Standard curve error value" was a LightCycler 480 standard curve parameter, and less than 0.2 was the acceptable range.

As shown in Table 2, the amplification efficiencies of the 8 primer-probe concentration combinations were all within the acceptable range of 90% to 110%. In the present invention, the preferred working concentration of OCT4-F-3 and OCT4-R-3 was 400nM, and the working concentration of OCT4-P-3 was 200nM.

3.1.2 Screening of NANOG gene primers and probes

NANOG candidate primers were as follows:

| Primer name | Sequence (5'-3') |
|---|---|
| NANOG-F-1 | CAGAAGGCCTCAGCACCTAC |
| NANOG-R-1 | TCCAGGTCTGGTTGCTCCAC |
| NANOG-F-2 | AACTCTCCAACATCCTGAACCT |
| NANOG-R-2 | CTGCGTCACACCATTGCTATT |
| NANOG-F-3 | ACCAGTCCCAAAGGCAAACA |
| NANOG-R-3 | TCTGCTGGAGGCTGAGGTAT |

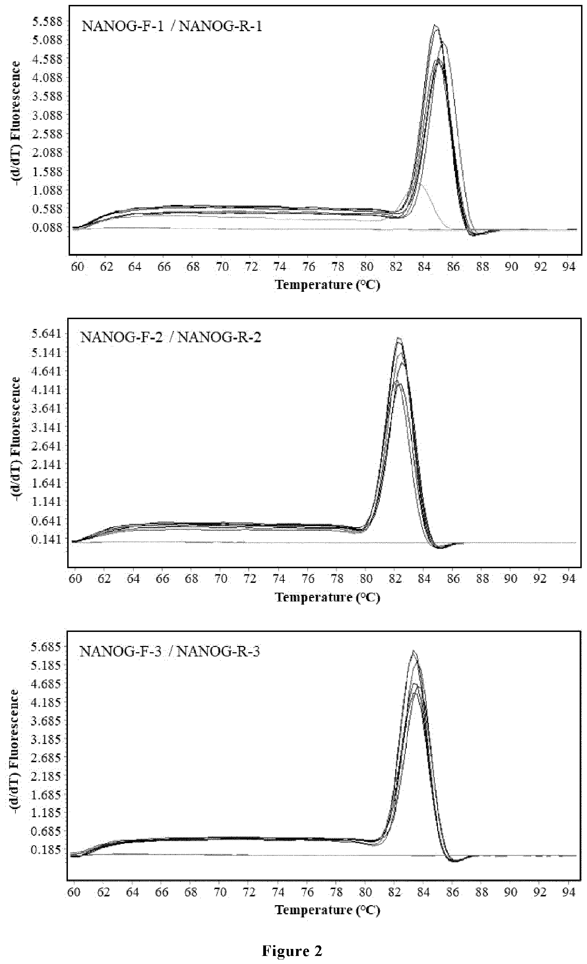

Using pNANOG plasmid as a template, three pairs of NANOG primers were screened for specificity using the dye method qPCR reagent HiScript® II One Step qRT-PCR SYBR® Green Kit. The results were shown in Figure 2.

As shown in Figure 2, the amplified products of the three primer pairs showed singlet dissolution peaks, and all met the requirements of specificity. In the present invention, NANOG-F-3/NANOG-R-3 was preferred for probe design and screening.

NANOG candidate probes were as follows:

| Probe name | Sequence (5'-3') | Probe label |
|---|---|---|
| NANOG-P-1 | AACAGGTGAAGACCTGGTTCCAGAACC | FAM-BHQ1 |
| NANOG-P-2 | CGGCCAGTTGTTTTTCTGCCACCTCT | FAM-BHQ1 |

Using pNANOG plasmid as a template, the two NANOG probes were tested using the probe method qPCR reagent HiScript® II U+ One Step qRT-PCR Probe Kit for amplification efficiency. The results were shown in Table 3.

Table 3: NANOG probe amplification efficiency test results

| Probe name | Amplification efficiency | Standard curve error value |
|---|---|---|
| NANOG-P-1 | 96.2% | 0.0344 |

(continued)

| Probe name | Amplification efficiency | Standard curve error value |
|---|---|---|
| NANOG-P-2 | 102.0% | 0.0216 |

Note: "Standard curve error value" was a LightCycler 480 standard curve parameter, and less than 0.2 was the acceptable range.

As shown in Table 3, the amplification efficiencies of NANOG-P-1 and NANOGA-P-2 were both within the acceptable range of 90% to 110%. In the present invention, the primer-probe combination NANOG-F-3/NANOG-R-3/NANOG-P-2 was preferred for optimization of working concentration.

The working concentration ranges and detection results of NANOG primers and probes were shown in Table 4.

Table 4: Amplification efficiency of NANOG primers and probes at different working concentrations

| No. | NANOG-F-3 Working concentration (nM) | NANOG-R-3 Working concentration (nM) | NANOG-P-2 Working concentration (nM) | Amplification efficiency | Standard curve error value |
|---|---|---|---|---|---|
| 1 | 250 | 250 | 125 | 96.2% | 0.0580 |
| 2 | 250 | 250 | 200 | 101.5% | 0.0432 |
| 3 | 400 | 400 | 200 | 100.4% | 0.0565 |
| 4 | 400 | 400 | 300 | 94.1% | 0.0599 |
| 5 | 400 | 400 | 400 | 97.7% | 0.0277 |
| 6 | 500 | 500 | 250 | 100.7% | 0.0329 |
| 7 | 500 | 500 | 350 | 94.3% | 0.1240 |
| 8 | 500 | 500 | 500 | 102.5% | 0.0840 |

Note: "Standard curve error value" was a LightCycler 480 standard curve parameter, and less than 0.2 was the acceptable range.

As shown in Table 4, the amplification efficiencies of the 8 primer-probe concentration combinations were all within the acceptable range of 90% to 110%. In the present invention, the preferred working concentrations of NANOG-F-3 and NANOG-R-3 were 400nM, and the working concentration of NANOG-P-2 was 200nM.

3.1.3 Screening of GAPDH gene primers and probes

GAPDH candidate primers were as follows:

| Primer name | Sequence (5'-3') |
|---|---|
| GAPDH-F-1 | GAAGGTGAAGGTCGGAGTC |
| GAPDH-R-1 | GAAGATGGTGATGGGATTTC |
| GAPDH-F-2 | ACCCACTCCTCCACCTTTGAC |
| GAPDH-R-2 | TGTTGCTGTAGCCAAATTCGTT |
| GAPDH-F-3 | GTCTCCTCTGACTTCAACAGCG |
| GAPDH-R-3 | ACCACCCTGTTGCTGTAGCCAA |

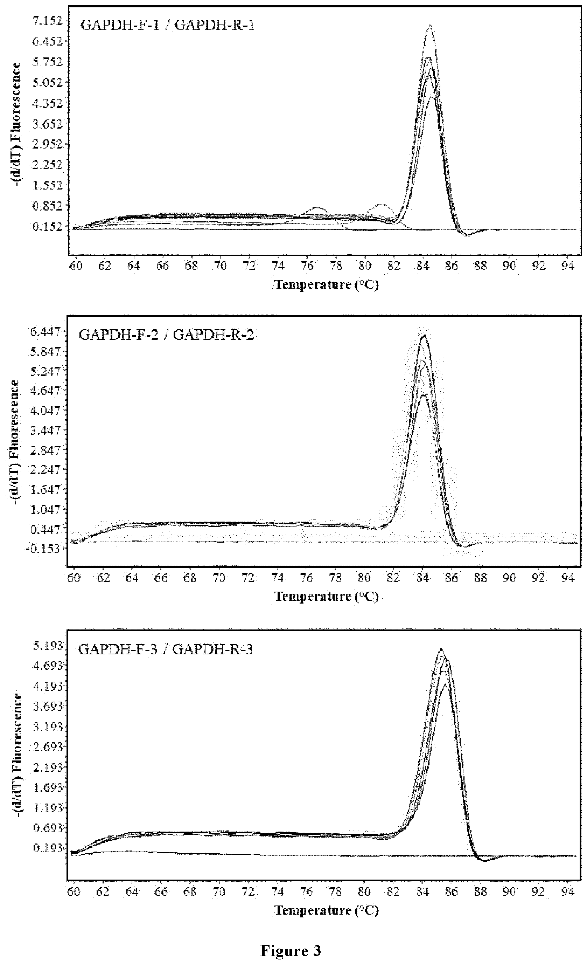

Using pGAPDH plasmid as a template, the three pairs of GAPDH primers were screened for specificity using the dye method qPCR reagent HiScript® II One Step qRT-PCR SYBR® Green Kit. The results were shown in Figure 3.

As shown in Figure 3, the amplification products of the primers GAPDH-F-2/GAPDH-R-2 and GAPDH-F-3/GAPDH-R-3 showed singlet dissolution peaks, and met the requirements of specificity. In the present invention, GAPDH-F-3/GAPDH-R-3 was preferred for probe design and screening.

GAPDH candidate probes were as follows:

| Probe name | Sequence (5'-3') | Probe label |
|---|---|---|
| GAPDH-P-1 | TGCCCTCAACGACCACTTTGTCAAGCT | FAM-BHQ1 |

(continued)

| Probe name | Sequence (5'-3') | Probe label |
|---|---|---|
| GAPDH-P-2 | CCTCCACCTTTGACGCTGGGGCTGGCA | FAM-BHQ1 |
| GAPDH-P-3 | TGGCATTGCCCTCAACGACCACTTTGT | FAM-BHQ1 |

Using pGAPDH plasmid as a template, the three GAPDH probes was tested using the probe method qPCR reagent HiScript® II U+ One Step qRT-PCR Probe Kit for amplification efficiency. The results were shown in Table 5.

Table 5: GAPDH probe amplification efficiency test results

| Probe name | Amplification efficiency | Standard curve error value |
|---|---|---|
| GAPDH-P-1 | 96.8% | 0.0752 |
| GAPDH-P-2 | 92.9% | 0.0466 |
| GAPDH-P-3 | 98.0% | 0.0408 |

Note: "Standard curve error value" was a LightCycler 480 standard curve parameter, and less than 0.2 was the acceptable range.

As shown in Table 5, the amplification efficiencies of the three GAPDH probes were within the acceptable range of 90% and 110%, wherein GAPDH-P-3 was the best overall, so the primer-probe combination GAPDH-F-3/GAPDH-R-3/GAPDH-P-3 was preferred for optimization of working concentrations.

The working concentration ranges and detection results of GAPDH primers and probes were shown in Table 6.

Table 6: Amplification efficiency of GAPDH primers and probes at different working concentrations

| No. | GAPDH-F-3 Working concentration (nM) | GAPDH-R-3 Working concentration (nM) | GAPDH-P-3 Working concentration (nM) | Amplification efficiency | Standard curve error value |
|---|---|---|---|---|---|
| 1 | 250 | 250 | 125 | 101.1% | 0.0480 |
| 2 | 250 | 250 | 200 | 93.1% | 0.1670 |
| 3 | 400 | 400 | 200 | 98.5% | 0.0247 |
| 4 | 400 | 400 | 300 | 101.8% | 0.0351 |
| 5 | 400 | 400 | 400 | 97.7% | 0.0425 |
| 6 | 500 | 500 | 250 | 98.3% | 0.0463 |
| 7 | 500 | 500 | 350 | 98.6% | 0.0269 |
| 8 | 500 | 500 | 500 | 98.3% | 0.0239 |

Note: "Standard curve error value" was a LightCycler 480 standard curve parameter, and less than 0.2 was the acceptable range.

As shown in Table 6, the amplification efficiencies of the 8 primer-probe concentration combinations were all within the acceptable range of 90% to 110%. In the present invention, the preferred working concentrations of GAPDH-F-3 and GAPDH-R-3 were 400nM, and the working concentration of GAPDH-P-3 was 200nM.

3.2 Reference product cell screening

The reference cells used in the method for testing pluripotency gene expression level should exhibit low and stable gene expression level. Human foreskin fibroblasts (HFF), human skin fibroblasts (HSF), bone marrow mesenchymal stem cells (BMMSC), adipose mesenchymal stem cells (ADMSC), umbilical cord mesenchymal stem cells (UCMSC) and human primary preadipocytes, human cerebral vascular pericytes, human chondrocytes, human primary aortic smooth muscle cells, and human primary osteoblasts were utilized to evaluate the expression levels of pluripotency genes OCT4 and NANOG. The test results were shown in Table 7 and Table 8.

Table 7: Detection results of OCT4 gene relative expression level in cells

| Sample name | Gene | Cp | | | | ΔCp |
|---|---|---|---|---|---|---|
| | | 1 | 2 | 3 | Mean | |
| HSF | GAPDH | 16.84 | 16.78 | 16.83 | 16.82 | 13.17 |
| | OCT4 | 30.26 | 29.85 | 29.85 | 29.99 | |
| HFF | GAPDH | 16.39 | 16.55 | 16.47 | 16.47 | 13.32 |
| | OCT4 | 29.70 | 29.77 | 29.89 | 29.79 | |
| ADMSC | GAPDH | 16.20 | 15.22 | 16.28 | 15.90 | 14.41 |
| | OCT4 | 30.38 | 30.23 | 30.31 | 30.31 | |
| BMMSC | GAPDH | 16.52 | 16.28 | 16.71 | 16.50 | 13.30 |
| | OCT4 | 29.94 | 29.86 | 29.60 | 29.80 | |
| UCMSC | GAPDH | 16.04 | 16.06 | 16.18 | 16.09 | 13.86 |
| | OCT4 | 30.01 | 29.81 | 30.03 | 29.95 | |
| Human primary preadipocyte | GAPDH | 16.10 | 16.15 | 16.12 | 16.12 | 13.70 |
| | OCT4 | 30.07 | 29.68 | 29.70 | 29.82 | |
| Human cerebral vascular pericyte | GAPDH | 16.03 | 15.80 | 16.16 | 16.00 | 14.20 |
| | OCT4 | 30.14 | 30.27 | 30.18 | 30.20 | |
| Human chondrocyte | GAPDH | 16.20 | 16.11 | 16.06 | 16.12 | 11.76 |
| | OCT4 | 28.09 | 27.60 | 27.94 | 27.88 | |
| Human primary aortic smooth muscle cell | GAPDH | 16.14 | 16.28 | 16.31 | 16.24 | 13.84 |
| | OCT4 | 30.10 | 30.13 | 30.01 | 30.08 | |
| Human primary osteoblast | GAPDH | 16.85 | 16.78 | 16.93 | 16.85 | 11.82 |
| | OCT4 | 28.54 | 28.57 | 28.91 | 28.67 | |

Table 8: Detection results of NANOG gene expression level in mesenchymal stem cells

| Sample name | Gene | Cp | | | | ΔCp |
|---|---|---|---|---|---|---|
| | | 1 | 2 | 3 | Mean | |
| HSF | GAPDH | 16.84 | 16.78 | 16.83 | 16.82 | 17.17 |
| | NANOG | 34.40 | 33.78 | 33.80 | 33.99 | |
| HFF | GAPDH | 16.15 | 16.08 | 16.05 | 16.09 | 17.19 |
| | NANOG | 33.27 | 32.84 | 33.74 | 33.28 | |
| ADMSC | GAPDH | 15.72 | 15.93 | 15.66 | 15.77 | 16.91 |
| | NANOG | 32.90 | 32.64 | 32.51 | 32.68 | |
| BMMSC | GAPDH | 15.80 | 16.13 | 16.03 | 15.99 | 15.86 |
| | NANOG | 31.99 | 31.64 | 31.93 | 31.85 | |
| UCMSC | GAPDH | 15.29 | 15.62 | 15.79 | 15.57 | 17.83 |
| | NANOG | 33.12 | 33.72 | 33.36 | 33.40 | |
| Human primary preadipocyte | GAPDH | 16.08 | 16.18 | 15.99 | 16.08 | 16.14 |
| | NANOG | 32.28 | 32.05 | 32.34 | 32.22 | |
| Human cerebral vascular pericyte | GAPDH | 16.07 | 15.97 | 15.68 | 15.91 | 17.10 |
| | NANOG | 32.98 | 33.20 | 32.85 | 33.01 | |
| Human chondrocyte | GAPDH | 16.05 | 16.09 | 16.06 | 16.07 | 15.67 |
| | NANOG | 31.32 | 31.76 | 32.14 | 31.74 | |

(continued)

| Sample name | Gene | Cp | | | | ΔCp |
|---|---|---|---|---|---|---|
| | | 1 | 2 | 3 | Mean | |
| Human primary aortic smooth muscle cell | GAPDH | 16.13 | 15.98 | 15.92 | 16.01 | 16.78 |
| | NANOG | 32.96 | 32.96 | 32.46 | 32.79 | |
| Human primary osteoblast | GAPDH | 16.61 | 16.54 | 16.39 | 16.51 | 16.66 |
| | NANOG | 32.98 | 33.36 | 33.16 | 33.17 | |

The results indicated that the expression levels of the pluripotency genes OCT4 and NANOG in HSF cells, HFF cells, BMMSC, ADMSC, UCMSC, human primary preadipocytes, human cerebral vascular pericytes, human primary aortic smooth muscle cells, human chondrocytes and human primary osteoblasts were comparable, as evidenced by similar ΔCq values. Consequently, these cells all possess the potential to serve as reference cells in the method for assessing the expression levels of pluripotency gene.

3.3 Confirmation of reference cells

HFF cells and HSF cells could be purchased directly from ATCC, which were easy to obtain and had high industry recognition. HFF cells and HSF cells were selected for multiple tests to study the stability of pluripotency gene expression. The results were shown in Table 9 and Table 10.

Table 9: Detection results of OCT4 gene expression level in HFF and HSF cells

| Cell sample | Gene | Cp | | | | ΔCp |
|---|---|---|---|---|---|---|
| | | 1 | 2 | 3 | Mean | |
| HFF (Experiment 1) | GAPDH | 15.95 | 15.98 | 15.80 | 15.91 | 13.35 |
| | OCT4 | 29.45 | 29.18 | 29.15 | 29.26 | |
| HFF (Experiment 2) | GAPDH | 16.67 | 16.70 | 16.67 | 16.68 | 13.36 |
| | OCT4 | 30.14 | 29.76 | 30.21 | 30.04 | |
| HFF (Experiment 3) | GAPDH | 16.83 | 16.89 | 16.83 | 16.85 | 12.52 |
| | OCT4 | 29.56 | 29.21 | 29.34 | 29.37 | |
| HSF (Experiment 1) | GAPDH | 16.84 | 16.78 | 16.83 | 16.82 | 13.17 |
| | OCT4 | 30.26 | 29.85 | 29.85 | 29.99 | |
| HSF (Experiment 2) | GAPDH | 16.88 | 16.56 | 16.74 | 16.73 | 13.34 |
| | OCT4 | 30.14 | 30.05 | 30.02 | 30.07 | |
| HSF (Experiment 3) | GAPDH | 16.79 | 16.89 | 16.90 | 16.86 | 10.69 |
| | OCT4 | 27.53 | 27.66 | 27.45 | 27.55 | |

Table 10: Detection results of NANOG gene expression level in HFF and HSF cells

| Cell sample | Gene | Cp | | | | ΔCp |
|---|---|---|---|---|---|---|
| | | 1 | 2 | 3 | Mean | |
| HFF (Experiment 1) | GAPDH | 15.95 | 15.98 | 15.80 | 15.91 | 16.60 |
| | NANOG | 32.02 | 32.65 | 32.85 | 32.51 | |
| HFF cell (Experiment 2) | GAPDH | 16.67 | 16.70 | 16.67 | 16.68 | 16.35 |
| | NANOG | 33.15 | 33.11 | 32.84 | 33.03 | |
| HFF | GAPDH | 16.28 | 16.23 | 16.18 | 16.23 | 16.23 |
| (Experiment 3) | NANOG | 32.81 | 32.29 | 32.29 | 32.46 | |

(continued)

| Cell sample | Gene | Cp | | | | ΔCp |
| --- | --- | --- | --- | --- | --- | --- |
| | | 1 | 2 | 3 | Mean | |
| HSF (Experiment 1) | GAPDH | 16.84 | 16.78 | 16.83 | 16.82 | 17.17 |
| | NANOG | 34.40 | 33.78 | 33.80 | 33.99 | |
| HSF (Experiment 2) | GAPDH | 16.88 | 16.56 | 16.74 | 16.73 | 16.85 |
| | NANOG | 33.34 | 33.22 | 34.19 | 33.58 | |
| HSF (Experiment 3) | GAPDH | 16.79 | 16.89 | 16.90 | 16.86 | 12.47 |
| | NANOG | 29.49 | 29.25 | 29.24 | 29.33 | |

It could be seen from Table 9 and 10 that in multiple experiments, the OCT4 and NANOG genes demonstrated high ΔCp stability in HFF cells, making HFF the reference cell for testing the expression levels of pluripotency genes. Other cell types, such as BMMSC, ADMSC, UCMSC, human primary preadipocyte, human cerebral vascular pericyte, human primary aortic smooth muscle cell, human chondrocyte, human primary osteoblast, etc., could also be considered as candidates for reference cells.

Example 4: Application

4.1 Testing expression levels of OCT4 and NANOG genes in pluripotent stem cells

Using HFF cells as reference cells, the relative expression levels of pluripotency genes OCT4 and NANOG in different batches of hESC and hiPSC were tested. The results were shown in Table 11 to Table 14.

Table 11: Test results of OCT4 gene expression level in hESC

| Sample and generation | Gene | Cp | | | | ΔCp | ΔΔCp | $2^{-\Delta\Delta Cp}$ |
| --- | --- | --- | --- | --- | --- | --- | --- | --- |
| | | 1 | 2 | 3 | Mean | | | |
| HFF | GAPDH | 15.84 | 15.48 | 15.63 | 15.65 | 12.17 | - | - |
| | OCT4 | 27.97 | 27.74 | 27.76 | 27.82 | | | |
| hESC (P30) | GAPDH | 15.91 | 15.90 | 15.89 | 15.90 | 0.89 | -11.28 | 2486.67 |
| | OCT4 | 16.70 | 16.93 | 16.74 | 16.79 | | | |
| hESC (P34) | GAPDH | 15.96 | 15.78 | 15.33 | 15.69 | 1.00 | -11.17 | 2304.12 |
| | OCT4 | 16.71 | 16.74 | 16.61 | 16.69 | | | |
| hESC (P37) | GAPDH | 15.71 | 15.99 | 15.20 | 15.63 | 1.29 | -10.88 | 1884.54 |
| | OCT4 | 16.73 | 16.85 | 17.17 | 16.92 | | | |
| hESC (P40) | GAPDH | 15.22 | 15.53 | 16.08 | 15.61 | 1.30 | -10.87 | 1871.53 |
| | OCT4 | 17.21 | 16.25 | 17.27 | 16.91 | | | |

Table 12: Test results of NANOG gene expression level in hESC

| Sample and generation | Gene | Cp | | | | ΔCp | ΔΔCp | $2^{-\Delta\Delta Cp}$ |
| --- | --- | --- | --- | --- | --- | --- | --- | --- |
| | | 1 | 2 | 3 | Mean | | | |
| HFF | GAPDH | 16.21 | 16.24 | 16.30 | 16.25 | 16.25 | - | - |
| | OCT4 | 32.47 | 32.53 | 32.51 | 32.50 | | | |
| hESC (P30) | GAPDH | 16.36 | 16.47 | 16.20 | 16.34 | 5.27 | -10.98 | 2019.80 |
| | OCT4 | 21.65 | 21.63 | 21.56 | 21.61 | | | |
| hESC (P34) | GAPDH | 16.24 | 16.27 | 16.22 | 16.24 | 4.99 | -11.26 | 2452.44 |
| | OCT4 | 21.17 | 21.18 | 21.33 | 21.23 | | | |

(continued)

| Sample and generation | Gene | Cp | | | | ΔCp | ΔΔCp | $2^{-\Delta\Delta Cp}$ |
|---|---|---|---|---|---|---|---|---|
| | | 1 | 2 | 3 | Mean | | | |
| hESC (P37) | GAPDH | 16.53 | 16.29 | 15.67 | 16.16 | 5.23 | -11.02 | 2076.59 |
| | OCT4 | 21.26 | 21.39 | 21.51 | 21.39 | | | |
| hESC (P40) | GAPDH | 16.69 | 16.55 | 16.55 | 16.60 | 4.94 | -11.31 | 2538.92 |
| | OCT4 | 21.46 | 21.41 | 21.75 | 21.54 | | | |

Table 13: Test results of OCT4 gene expression level in hiPSC

| Sample and generation | Gene | Cp | | | | ΔCp | ΔΔCp | $2^{-\Delta\Delta Cp}$ |
|---|---|---|---|---|---|---|---|---|
| | | 1 | 2 | 3 | Mean | | | |
| HFF | GAPDH | 15.95 | 15.98 | 15.80 | 15.91 | 13.35 | - | - |
| | OCT4 | 29.45 | 29.18 | 29.15 | 29.26 | | | |
| iPSC (P19) | GAPDH | 17.38 | 17.14 | 17.35 | 17.29 | 2.05 | -11.30 | 2521.38 |
| | OCT4 | 19.31 | 19.42 | 19.28 | 19.34 | | | |
| iPSC (P29) | GAPDH | 16.13 | 15.94 | 16.30 | 16.12 | 2.03 | -11.32 | 2556.58 |
| | OCT4 | 18.16 | 18.16 | 18.13 | 18.15 | | | |

Table 14: Test results of NANOG gene expression level in hiPSC

| Sample and generation | Gene | Cp | | | | ΔCp | ΔΔCp | $2^{-\Delta\Delta Cp}$ |
|---|---|---|---|---|---|---|---|---|
| | | 1 | 2 | 3 | Mean | | | |
| HFF | GAPDH | 15.95 | 15.98 | 15.80 | 15.91 | 16.60 | - | - |
| | OCT4 | 32.02 | 32.65 | 32.85 | 32.51 | | | |
| iPSC (P19) | GAPDH | 17.38 | 17.14 | 17.35 | 17.29 | 5.25 | -11.35 | 2610.30 |
| | OCT4 | 22.54 | 22.54 | 22.40 | 22.54 | | | |
| iPSC (P29) | GAPDH | 16.13 | 15.94 | 16.30 | 16.12 | 5.47 | -11.13 | 2241.11 |
| | OCT4 | 21.62 | 21.43 | 21.72 | 21.59 | | | |

The results showed that in hESCs of P30, P34, P37, and P40 generations, the relative expression levels of OCT4 gene were from 1871.53 to 2486.67, and the relative expression levels of NANOG gene were from 2019.80 to 2538.93. The pluripotency gene expression levels of hESCs in different generations were stable.

The results showed that in hiPSCs of P19 and P29 passages, the relative expression levels of OCT4 gene were from 2521.38 to 2556.58, and the relative expression levels of NANOG gene were from 2241.11 to 2610.30. The expression levels of pluripotency genes in hiPSCs of different passages were stable and comparable to the expression levels of hESCs.

Therefore, this detection method could be utilized for assessing the expression levels of pluripotency genes in hPSCs, as well as for investigating the stability of pluripotency gene expression across different generations of hPSCs. It is also applicable for testing various cell banks, including hESCs and iPSCs, with consistent results.

4.2 Detection of hESC residue

For cell therapy products derived from pluripotent stem cells, tumorigenicity must be considered. The International Society for Cell Therapy (ISCT) has emphasized that the essence of tumorigenicity testing of hPSC-derived cells is the detection of residual undifferentiated cells. However, currently, no standard method for testing hPSC residues had been established in the industry[1]. In the present invention, HFF cells were used as reference cells to detect the relative expression levels of pluripotency genes OCT4 and NANOG in different batches of hESC-derived mesenchymal-like stem cells, and to determine the level of hESCs residue therein. The test results were shown in Table 15 and Table 16.

Table 15: Test results of OCT4 gene expression level in mesenchymal-like stem cells

| Sample and batch | Gene | Cp | | | | ΔCp | ΔΔCp | $2^{-\Delta\Delta Cp}$ |
|---|---|---|---|---|---|---|---|---|
| | | 1 | 2 | 3 | Mean | | | |
| HFF | GAPDH | 16.67 | 16.70 | 16.67 | 16.68 | 13.36 | -- | -- |
| | OCT4 | 30.14 | 29.76 | 30.21 | 30.04 | | | |
| Mesenchymal-like stem cell Y202002002 | GAPDH | 17.08 | 16.98 | 17.02 | 17.03 | 13.77 | 0.41 | 0.75 |
| | OCT4 | 30.75 | 30.81 | 30.85 | 30.80 | | | |
| Mesenchymal-like stem cell Y202007009 | GAPDH | 16.97 | 17.07 | 17.07 | 17.04 | 13.38 | 0.02 | 0.99 |
| | OCT4 | 30.39 | 25.02 | 30.45 | 30.42 | | | |
| Mesenchymal-like stem cell Y202009010 | GAPDH | 17.08 | 17.03 | 16.97 | 17.03 | 12.96 | -0.40 | 1.32 |
| | OCT4 | 30.06 | 29.97 | 29.93 | 29.99 | | | |

Table 16: Test results of NANOG gene expression level in mesenchymal-like stem cells

| Sample and batch | Gene | Cp | | | | ΔCp | ΔΔCp | $2^{-\Delta\Delta Cp}$ |
|---|---|---|---|---|---|---|---|---|
| | | 1 | 2 | 3 | Mean | | | |
| HFF | GAPDH | 16.67 | 16.70 | 16.67 | 16.68 | 16.35 | -- | -- |
| | OCT4 | 33.15 | 33.11 | 32.84 | 33.03 | | | |
| Mesenchymal-like stem cell Y202002002 | GAPDH | 17.08 | 16.98 | 17.02 | 17.03 | 16.39 | 0.04 | 0.97 |
| | OCT4 | 33.52 | 33.22 | 33.52 | 33.42 | | | |
| Mesenchymal-like stem cell Y202007009 | GAPDH | 16.97 | 17.07 | 17.07 | 17.04 | 15.92 | -0.43 | 1.35 |
| | OCT4 | 33.37 | 32.85 | 32.66 | 32.96 | | | |
| Mesenchymal-like stem cell Y202009010 | GAPDH | 17.08 | 17.03 | 16.97 | 17.03 | 15.53 | -0.82 | 1.77 |
| | OCT4 | 32.91 | 32.40 | 32.38 | 32.56 | | | |

It could be seen from Table 15 and Table 16 that in different batches of mesenchymal-like stem cells, the relative expression levels of OCT4 gene were from 0.75 to 1.32, and the relative expression levels of NANOG gene were from 0.97 to 1.77, which were comparable to the expression level in the reference cell HFF. This showed that there were no hESCs residues in the mesenchymal stem-like cells. Therefore, the above detection method could be used for hESCs residue detection. This detection method could quickly detect hPSCs residues in hESCs or hiPSC-derived products and had a sensitivity of 0.01%, as shown below.

4.3 Study on the process of directional differentiation of pluripotent stem cells

Using HFF cells as reference cells, the relative expression levels of pluripotency genes OCT4 and NANOG in P0, P1, P2, P3, P4, and P5 of the cells during directional differentiation process from hESCs were detected to characterize the differentiation process of pluripotent stem cells. The test results were shown in Table 17 and Table 18.

Table 17: Test results of OCT4 gene expression level in cells during directional differentiation process from hESCs

| Sample and generation | Gene | Cp | | | | ΔCp | ΔΔCp | $2^{-\Delta\Delta Cp}$ |
|---|---|---|---|---|---|---|---|---|
| | | 1 | 2 | 3 | Mean | | | |
| HFF | GAPDH | 16.83 | 16.89 | 16.83 | 16.85 | 12.52 | -- | -- |
| | OCT4 | 29.56 | 29.21 | 29.34 | 29.37 | | | |
| Differentiated cells P0 generation | GAPDH | 16.77 | 16.97 | 16.75 | 16.83 | 4.09 | -8.43 | 344.89 |
| | OCT4 | 20.79 | 21.08 | 20.89 | 20.92 | | | |
| Differentiated cells P1 generation | GAPDH | 16.74 | 16.90 | 16.70 | 16.78 | 7.96 | -4.56 | 23.59 |
| | OCT4 | 24.78 | 24.70 | 24.74 | 24.74 | | | |

(continued)

| Sample and generation | Gene | Cp | | | | $\Delta$Cp | $\Delta\Delta$Cp | $2^{-\Delta\Delta Cp}$ |
|---|---|---|---|---|---|---|---|---|
| | | 1 | 2 | 3 | Mean | | | |
| Differentiated cells P2 generation | GAPDH | 16.80 | 16.94 | 16.78 | 16.84 | 11.70 | -0.82 | 1.77 |
| | OCT4 | 28.60 | 28.65 | 28.37 | 28.54 | | | |
| Differentiated cells P3 generation | GAPDH | 16.87 | 17.12 | 16.70 | 16.90 | 12.50 | -0.02 | 1.01 |
| | OCT4 | 29.36 | 29.49 | 29.34 | 29.40 | | | |
| Differentiated cells P4 generation | GAPDH | 16.68 | 16.81 | 16.67 | 16.72 | 13.88 | 1.36 | 0.39 |
| | OCT4 | 30.61 | 30.47 | 30.71 | 30.60 | | | |
| Differentiated cells P5 generation | GAPDH | 17.05 | 16.85 | 17.04 | 16.98 | 12.68 | 0.16 | 0.90 |
| | OCT4 | 29.78 | 29.56 | 29.64 | 29.66 | | | |

Table 18: Test results of NANOG gene expression level in cells during directional differentiation process from hESCs

| Sample and generation | Gene | Cp | | | | $\Delta$Cp | $\Delta\Delta$Cp | $2^{-\Delta\Delta Cp}$ |
|---|---|---|---|---|---|---|---|---|
| | | 1 | 2 | 3 | Mean | | | |
| HFF | GAPDH | 16.28 | 16.23 | 16.18 | 16.23 | 16.23 | -- | -- |
| | NANOG | 32.81 | 32.29 | 32.29 | 32.46 | | | |
| Differentiated cells P0 generation | GAPDH | 16.01 | 16.52 | 16.44 | 16.32 | 7.17 | -9.06 | 533.74 |
| | NANOG | 23.57 | 23.39 | 23.50 | 23.49 | | | |
| Differentiated cells P1 generation | GAPDH | 16.32 | 16.52 | 16.46 | 16.43 | 10.96 | -5.27 | 38.59 |
| | NANOG | 27.68 | 27.03 | 27.47 | 27.39 | | | |
| Differentiated cells P2 generation | GAPDH | 16.31 | 16.51 | 16.39 | 16.40 | 14.54 | -1.69 | 3.23 |
| | NANOG | 30.79 | 30.88 | 31.16 | 30.94 | | | |
| Differentiated cells P3 generation | GAPDH | 16.57 | 16.41 | 16.40 | 16.46 | 15.58 | -0.65 | 1.57 |
| | NANOG | 31.97 | 31.91 | 32.24 | 32.04 | | | |
| Differentiated cells P4 generation | GAPDH | 16.37 | 16.60 | 16.48 | 16.48 | 17.03 | 0.80 | 0.57 |
| | NANOG | 33.06 | 33.96 | 33.51 | 33.51 | | | |
| Differentiated cells P5 generation | GAPDH | 16.61 | 16.37 | 16.72 | 16.57 | 15.92 | -0.31 | 1.24 |
| | NANOG | 32.04 | 32.68 | 32.74 | 32.49 | | | |

As shown in Table 17 and Table 18, the relative expression levels of OCT4 gene and NANOG gene gradually decreased during the directional differentiation of hESCs, and the expression levels of P3 to P5 tended to be stable. Therefore, this detection method could be used to characterize the directional differentiation process of hESCs.

4.4 Sensitivity of pluripotency gene detection method

To determine the sensitivity of the detection method, 10%, 1%, 0.1%, and 0.01% hESC were added to the mesenchymal stem cells differentiated from hESCs, and HFF cells were used as reference cells. The relative expression levels of pluripotency genes OCT4 and NANOG were detected in each cell sample. The test results were shown in Table 19 and Table 20.

Table 19: Sensitivity of method for testing pluripotency gene OCT4 expression level

| Sample name | Gene | Cp | | | | $\Delta$Cp | $\Delta\Delta$Cp | $2^{-\Delta\Delta Cp}$ |
|---|---|---|---|---|---|---|---|---|
| | | 1 | 2 | 3 | Mean | | | |
| HFF | GAPDH | 16.68 | 16.76 | 16.46 | 16.63 | 12.92 | -- | -- |
| | OCT4 | 29.60 | 29.45 | 29.60 | 29.55 | | | |

(continued)

| Sample name | Gene | Cp | | | | ΔCp | ΔΔCp | $2^{-\Delta\Delta Cp}$ |
|---|---|---|---|---|---|---|---|---|
| | | 1 | 2 | 3 | Mean | | | |
| hESC | GAPDH | 16.29 | 16.38 | 16.09 | 16.25 | 1.31 | -11.61 | 3125.78 |
| | OCT4 | 17.66 | 17.63 | 17.40 | 17.56 | | | |
| Differentiated cells P5 generation + 10% hESC | GAPDH | 16.88 | 16.95 | 17.17 | 17.00 | 3.53 | -9.39 | 670.92 |
| | OCT4 | 20.48 | 20.66 | 20.44 | 20.53 | | | |
| Differentiated cells P5 generation + 1% hESC | GAPDH | 16.89 | 16.90 | 17.03 | 16.94 | 7.04 | -5.88 | 58.89 |
| | OCT4 | 24.02 | 23.89 | 24.03 | 23.98 | | | |
| Differentiated cells P5 generation + 0.1% hESC | GAPDH | 17.07 | 16.93 | 16.82 | 16.94 | 10.29 | -2.63 | 6.19 |
| | OCT4 | 27.30 | 27.14 | 27.24 | 27.23 | | | |
| Differentiated cells P5 generation + 0.01% hESC | GAPDH | 16.93 | 16.88 | 17.19 | 17.00 | 12.53 | -0.39 | 1.31 |
| | OCT4 | 29.36 | 29.76 | 29.47 | 29.53 | | | |
| Differentiated cells P5 generation | GAPDH | 16.80 | 16.80 | 16.74 | 16.78 | 13.31 | 0.39 | 0.76 |
| | OCT4 | 29.87 | 30.38 | 30.03 | 30.09 | | | |

Table 20: Sensitivity of method for testing pluripotency gene NANOG expression level

| Sample name | Gene | Cp | | | | ΔCp | ΔΔCp | $2^{-\Delta\Delta Cp}$ |
|---|---|---|---|---|---|---|---|---|
| | | 1 | 2 | 3 | Mean | | | |
| HFF | GAPDH | 16.32 | 16.20 | 16.36 | 16.29 | 16.82 | - | - |
| | NANOG | 33.16 | 33.28 | 32.89 | 33.11 | | | |
| hESC | GAPDH | 15.88 | 16.07 | 15.81 | 15.92 | 5.77 | -11.05 | 2120.22 |
| | NANOG | 21.71 | 21.69 | 21.66 | 21.69 | | | |
| Differentiated cells P5 generation + 10% hESC | GAPDH | 16.71 | 16.60 | 16.48 | 16.60 | 7.81 | -9.01 | 515.56 |
| | NANOG | 24.60 | 24.31 | 24.32 | 24.41 | | | |
| Differentiated cells P5 generation + 1% hESC | GAPDH | 16.56 | 16.45 | 16.65 | 16.55 | 11.38 | -5.44 | 43.41 |
| | NANOG | 27.94 | 27.89 | 27.96 | 27.93 | | | |
| Differentiated | GAPDH | 16.17 | 16.17 | 16.26 | 16.20 | 14.75 | -2.07 | 4.20 |
| cells P5 generation + 0.1% hESC | NANOG | 30.99 | 30.76 | 31.10 | 30.95 | | | |
| Differentiated cells P5 generation + 0.01% hESC | GAPDH | 16.32 | 17.01 | 16.48 | 16.60 | 15.45 | -1.37 | 2.58 |
| | NANOG | 32.33 | 31.79 | 32.03 | 32.05 | | | |
| Differentiated cells P5 generation | GAPDH | 16.26 | 16.31 | 16.14 | 16.24 | 15.89 | -0.93 | 1.91 |
| | NANOG | 32.30 | 32.11 | 31.99 | 32.13 | | | |

As shown in Table 19 and Table 20, when 0.01% hESCs were added to the mesenchymal stem cells differentiated from hESCs, the relative expression level of the OCT4 gene was 1.31, which was greater than that of the cell sample without adding hESCs (the relative expression level was 0.76); the relative expression level of the NANOG gene was 2.58, which was greater than the cell sample without adding hESC (relative expression level was 1.91). Therefore, when this detection method was used for hESC residue detection, the sensitivity could reach 0.01%.

4.5 Intermediate precision study

Using HFF cells as reference cells, the relative expression levels of hESC pluripotency genes OCT4 and NANOG were detected at three different times, and three hESC samples were detected at each time, to study the intermediate precision of the detection method. The test results were shown in Table 21 and Table 22.

Table 21: Intermediate precision of method for testing pluripotency gene OCT4 expression level

| Experiment | Cell name | | Gene | Cp | | | | ΔCp | -ΔΔCp |
|---|---|---|---|---|---|---|---|---|---|
| | | | | 1 | 2 | 3 | Mean | | |
| Time 1 | HFF cell | | GAPDH | 15.92 | 15.95 | 16.20 | 16.02 | 13.12 | - |
| | | | OCT4 | 29.22 | 29.06 | 29.15 | 29.14 | | |
| | hESC | 1 | GAPDH | 16.02 | 15.83 | 16.09 | 15.98 | 1.17 | 11.95 |
| | | | OCT4 | 16.53 | 17.27 | 17.65 | 17.15 | | |
| | | 2 | GAPDH | 15.82 | 15.88 | 15.88 | 15.86 | 1.27 | 11.85 |
| | | | OCT4 | 17.13 | 17.10 | 17.16 | 17.13 | | |
| | | 3 | GAPDH | 15.15 | 15.68 | 15.66 | 15.50 | 1.56 | 11.56 |
| | | | OCT4 | 16.96 | 17.19 | 17.04 | 17.06 | | |
| Time 2 | HFF cell | | GAPDH | 16.53 | 16.42 | 16.34 | 16.43 | 12.27 | - |
| | | | OCT4 | 28.64 | 28.63 | 28.83 | 28.70 | | |
| | hESC | 1 | GAPDH | 16.76 | 16.54 | 16.80 | 16.70 | 0.78 | 11.49 |
| | | | OCT4 | 17.69 | 17.44 | 17.32 | 17.48 | | |
| | | 2 | GAPDH | 16.53 | 16.40 | 16.70 | 16.54 | 1.04 | 11.23 |
| | | | OCT4 | 17.58 | 17.57 | 17.58 | 17.58 | | |
| | | 3 | GAPDH | 16.44 | 16.44 | 16.55 | 16.48 | 1.05 | 11.22 |
| | | | OCT4 | 17.47 | 17.63 | 17.48 | 17.53 | | |
| Time 3 | HFF cell | | GAPDH | 16.17 | 16.29 | 16.24 | 16.23 | 13.36 | - |
| | | | OCT4 | 29.44 | 29.63 | 29.71 | 29.59 | | |
| | hESC | 1 | GAPDH | 16.57 | 16.44 | 16.57 | 16.53 | 1.00 | 12.36 |
| | | | OCT4 | 17.55 | 17.49 | 17.55 | 17.53 | | |
| | | 2 | GAPDH | 16.53 | 16.58 | 16.70 | 16.60 | 0.97 | 12.39 |
| | | | OCT4 | 17.56 | 17.53 | 17.63 | 17.57 | | |
| | | 3 | GAPDH | 16.46 | 16.46 | 16.72 | 16.55 | 1.16 | 12.20 |
| | | | OCT4 | 17.57 | 17.68 | 17.88 | 17.71 | | |
| Result statistics | Mean | | | - | - | - | - | - | 11.81 |
| | SD | | | - | - | - | - | - | 0.46 |

Table 22: Intermediate precision of method for testing pluripotency gene NANOG expression level

| Experiment | Cell name | | Gene | Cp | | | | ΔCp | -ΔΔCp |
|---|---|---|---|---|---|---|---|---|---|
| | | | | 1 | 2 | 3 | Mean | | |
| Time 1 | HFF cell | | GAPDH | 16.22 | 16.13 | 16.15 | 16.17 | 16.02 | |
| | | | NANOG | 31.05 | 33.08 | 32.45 | 32.19 | | |
| | hESC | 1 | GAPDH | 16.26 | 16.04 | 16.00 | 16.10 | 5.07 | 10.95 |
| | | | NANOG | 21.19 | 21.20 | 21.12 | 21.17 | | |
| | | 2 | GAPDH | 16.21 | 16.06 | 16.06 | 16.11 | 4.98 | 11.04 |

(continued)

| Experiment | Cell name | | Gene | Cp | | | | ΔCp | -ΔΔCp |
| | | | | 1 | 2 | 3 | Mean | | |
|---|---|---|---|---|---|---|---|---|---|
| | | 3 | NANOG | 21.08 | 21.24 | 20.96 | 21.09 | | |
| | | | GAPDH | 16.06 | 15.31 | 16.06 | 15.81 | 4.83 | 11.19 |
| | | | NANOG | 21.03 | 20.99 | 19.91 | 20.64 | | |
| Time 2 | HFF cell | | GAPDH | 16.25 | 16.40 | 16.37 | 16.34 | 16.04 | - |
| | | | NANOG | 32.44 | 32.28 | 32.43 | 32.38 | | |
| | hESC | 1 | GAPDH | 16.45 | 16.30 | 16.35 | 16.37 | 4.93 | 11.11 |
| | | | NANOG | 21.41 | 21.06 | 21.42 | 21.30 | | |
| | | 2 | GAPDH | 16.39 | 16.26 | 16.40 | 16.35 | 4.98 | 11.06 |
| | | | NANOG | 21.23 | 21.42 | 21.35 | 21.33 | | |
| | | 3 | GAPDH | 16.29 | 16.31 | 16.36 | 16.32 | 4.91 | 11.13 |
| | | | NANOG | 21.32 | 21.12 | 21.24 | 21.23 | | |
| Time 3 | HFF cell | | GAPDH | 16.00 | 16.10 | 16.17 | 16.09 | 16.85 | - |
| | | | NANOG | 33.13 | 32.62 | 33.07 | 32.94 | | |
| | hESC | 1 | GAPDH | 16.16 | 16.20 | 15.94 | 16.10 | 5.13 | 11.72 |
| | | | NANOG | 21.37 | 21.18 | 21.14 | 21.23 | | |
| | | 2 | GAPDH | 16.20 | 16.16 | 16.26 | 16.21 | 5.10 | 11.75 |
| | | | NANOG | 21.35 | 21.26 | 21.32 | 21.31 | | |
| | | 3 | GAPDH | 16.21 | 16.06 | 16.09 | 16.12 | 5.14 | 11.71 |
| | | | NANOG | 21.26 | 21.33 | 21.19 | 21.26 | | |
| Result statistics | Mean | | | - | - | - | - | - | 11.30 |
| | SD | | | - | - | - | - | - | 0.33 |

As shown in Table 21 and Table 22 that the -ΔΔCp SD values of OCT4 and NANOG genes in the nine hESC samples tested at different times were all less than 1, and the results were relatively stable. Therefore, the intermediate precision study results of this detection method met the requirements.

Example 5: Other reference cells

Since human skin fibroblasts (HSF) and human gingival fibroblasts (HGF), as fibroblasts, also expressed a small amount of OCT4 and NANOG genes[2,3], replacing the reference cells with HGF and HSF cells could also provide stable detection results in expression levels.

In addition, since the expression levels of pluripotency genes OCT4 and NANOG in BMMSC, ADMSC, UCMSC, human primary preadipocytes, human cerebral vascular pericytes, human primary aortic smooth muscle cells, human primary osteoblasts and other cells were comparable to those of HFF cells, they could thus serve as reference cells.

References:

1. Sato Y, Bando H, Di Piazza M, et al. Tumorigenicity assessment of cell therapy products: The need for global consensus and points to consider. Cytotherapy 2019; 21(11): 1095-111.

2. Monterubbianesi R, Bencun M, Pagella P, Woloszyk A, Orsini G, Mitsiadis TA. A comparative in vitro study of the osteogenic and adipogenic potential of human dental pulp stem cells, gingival fibroblasts and foreskin fibroblasts.

Sci Rep 2019; 9(1): 1761.

3. Hambiliki F, Strom S, Zhang P, Stavreus-Evers A. Co-localization of NANOG and OCT4 in human pre-implantation embryos and in human embryonic stem cells. J Assist Reprod Genet 2012; 29(10): 1021-8.

Although illustrative examples have been shown and described herein, those skilled in the art will understand that the above-described examples should not be construed as limitations of the present invention, and may be changed, substituted and modified without departing from the spirit, principles and ranges of the present invention.

**Claims**

1. Use of a reference cell for testing the expression level of pluripotency genes, wherein the reference cell is selected from cells with low and stable expression level of a pluripotency gene, optionally, the reference cell is selected from the group consisting of human foreskin fibroblast (HFF), human skin fibroblast (HSF), bone marrow mesenchymal stem cell (BMMSC), adipose mesenchymal stem cell (ADMSC), umbilical cord mesenchymal stem cell (UCMSC), as well as human primary preadipocyte, human cerebral vascular pericyte, human chondrocyte, human primary aortic smooth muscle cell, human primary osteoblast, preferably, the reference cell is selected from HFF cell, optionally, the pluripotency gene is selected from OCT4 and NANOG.

2. The use according to claim 1, wherein testing pluripotency gene expression level comprises selecting an internal control, optionally, the internal control is selected from the group consisting of an internal positive control and an internal negative control, preferably, the internal positive control is selected from GAPDH gene.

3. The use according to claim 1 or 2, wherein the reference cell can be used to test the hPSC residue level in a human pluripotent stem cell (hPSC)-related preparation, or to characterize a hPSC differentiation process.

4. The use according to any one of claims 1 to 3, wherein the pluripotency gene expression level is quantified in the form of $2^{\Delta\Delta Cq}$.

5. An OCT4 gene detection agent, which comprises an OCT4 gene forward primer sequence, an OCT4 gene reverse primer sequence, and optionally an OCT4 gene probe sequence, optionally, the OCT4 gene forward primer sequence (5'-3') is AGGAAGCTGACAACAATGAA, the OCT4 gene reverse primer sequence (5'-3') is TTGCCTCTCACTCGGTTC, and the OCT4 gene probe sequence (5'-3') is FAM-TTCGCTTTCTCTTTCGGGCCTGCACG-BHQ1.

6. A NANOG gene detection agent, which comprises a NANOG gene forward primer sequence, a NANOG gene reverse primer sequence, and optionally a NANOG gene probe sequence, optionally, the NANOG gene forward primer sequence (5'-3') is AACTCTCCAACATCCTGAACCT, the NANOG gene reverse primer sequence (5'-3') is CTGCGTCACACCATTGCTATT, and the NANOG gene probe sequence (5'-3') is FAM-CGGCCAGTTGTTTTTCTGCCACCTCT-BHQ1.

7. A GAPDH gene detection agent, which comprises a GAPDH gene forward primer sequence, a GAPDH gene reverse primer sequence, and optionally a GAPDH gene probe sequence, optionally, the GAPDH gene forward primer sequence (5'-3') is GTCTCCTCTGACTTCAACAGCG, the GAPDH gene reverse primer sequence (5'-3') is ACCACCCTGTTGCTGTAGCCAA, and the GAPDH gene probe sequence (5'-3') is FAM-CCTCCACCTTTGACGCTGGGGCTGGCA-BHQ1.

8. A detection method for detecting the pluripotency gene expression levels, comprising:

   (a) providing a sample to be tested;
   (b) providing a reference cell, wherein the reference cell is selected from cells with low and stable expression level of a pluripotency gene, optionally, the reference cell is selected from the group consisting of human foreskin fibroblast (HFF), human skin fibroblast (HSF), bone marrow mesenchymal stem cell (BMMSC), adipose mesenchymal stem cell (ADMSC), umbilical cord mesenchymal stem cell (UCMSC), human primary preadipocyte, human cerebral vascular pericyte, human chondrocyte, human primary aortic smooth muscle cell, human primary osteoblast, preferably, the reference cell is selected from HFF cell;
   (c) extracting RNA from the sample to be detected;
   (d) testing the expression level of the pluripotency gene using OCT4 or NANOG as a test gene, and GAPDH as an internal reference gene;

(e) determining the pluripotency gene expression level of the sample to be tested by comparing the expression level of the test gene in the sample to be tested with the expression level of the test gene in the reference cell.

9. The detection method according to claim 8, wherein the RNA extraction process comprises two genome removal steps to ensure the effect of genome removal.

10. The detection method according to claim 8 or 9, wherein the method involves detecting the expression level of the pluripotency gene in the sample to be tested using RT-qPCR, optionally, the method includes a reverse transcriptase-free control (NRC) to ensure that the detection results for all genes in RT-qPCR are negative.

11. Use of the detection method according to any one of claims 8 to 10 for testing the residue level of hPSC and for characterizing the differentiation process of hPSC.

**Figure 1**

**Figure 2**

**Figure 3**

**Figure 4**

## INTERNATIONAL SEARCH REPORT

| International application No. |
|---|
| **PCT/CN2022/139965** |

**A.    CLASSIFICATION OF SUBJECT MATTER**

C12Q 1/6851(2018.01)i;C12N 15/11(2006.01)i

According to International Patent Classification (IPC) or to both national classification and IPC

**B.    FIELDS SEARCHED**

Minimum documentation searched (classification system followed by classification symbols)

C12Q,C12N

Documentation searched other than minimum documentation to the extent that such documents are included in the fields searched

Electronic data base consulted during the international search (name of data base and, where practicable, search terms used)

TWMED; TWTXT; USTXT; DWPI; ENTXTC; KRTXT; CNTXT; WPABSC; JPTXT; CNABS; EPTXT; CNMED; WPABS; ENTXT; WOTXT; CJFD; CATXT; VEN, pubmed, genbank, elsevier science, CNKI, 万方, WANFANG, genbank, 中国专利生物序列检索系统, Chinese Patent Biological Sequence Retrieval System: 包皮, foreskin, 成纤维细胞, fibroblast, HFF, 表达, expression, 对照, 对照细胞, control, 基因, gene, OCT4, OCT-4, NANOG, GAPDH, actin, 内参, 看家基因, 管家基因, housekeeping gene, △Ct, OCT4, NANOG和GAPDH相关的引物探针检索

**C.    DOCUMENTS CONSIDERED TO BE RELEVANT**

| Category* | Citation of document, with indication, where appropriate, of the relevant passages | Relevant to claim No. |
|---|---|---|
| X | US 2012288936 A1 (AHLFORS JAN-ERIC et al.) 15 November 2012 (2012-11-15) see description, embodiment 1, paragraph 272, embodiments 2-4 and 28, and table 8c | 1-11 |
| A | US 2017313978 A1 (UNIVERSITY OF BATH et al.) 02 November 2017 (2017-11-02) see abstract, and embodiment 1 | 1-11 |
| A | WO 2007079533 A1 (SOUTH EASTERN SYDNEY AND ILLAW; SIDHU KULDIP S; TUCH BERNARD E;) 19 July 2007 (2007-07-19) see abstract, claims 1-20, embodiments 1-3, and figure 2 | 1-11 |
| X | CN 107988329 A (GUANGDONG XTEM BIOTECHNOLOGY CO., LTD.) 04 May 2018 (2018-05-04) see abstract, and embodiment 1 | 1-11 |

☑ Further documents are listed in the continuation of Box C.          ☑ See patent family annex.

| | |
|---|---|
| *    Special categories of cited documents: <br> "A"  document defining the general state of the art which is not considered to be of particular relevance <br> "D"  document cited by the applicant in the international application <br> "E"  earlier application or patent but published on or after the international filing date <br> "L"  document which may throw doubts on priority claim(s) or which is cited to establish the publication date of another citation or other special reason (as specified) <br> "O"  document referring to an oral disclosure, use, exhibition or other means <br> "P"  document published prior to the international filing date but later than the priority date claimed | "T"  later document published after the international filing date or priority date and not in conflict with the application but cited to understand the principle or theory underlying the invention <br> "X"  document of particular relevance; the claimed invention cannot be considered novel or cannot be considered to involve an inventive step when the document is taken alone <br> "Y"  document of particular relevance; the claimed invention cannot be considered to involve an inventive step when the document is combined with one or more other such documents, such combination being obvious to a person skilled in the art <br> "&"  document member of the same patent family |

| Date of the actual completion of the international search | Date of mailing of the international search report |
|---|---|
| **09 March 2023** | **16 March 2023** |

| Name and mailing address of the ISA/CN | Authorized officer |
|---|---|
| **China National Intellectual Property Administration (ISA/ CN)** <br> **China No. 6, Xitucheng Road, Jimenqiao, Haidian District, Beijing 100088** | |
| Facsimile No. **(86-10)62019451** | Telephone No. |

Form PCT/ISA/210 (second sheet) (July 2022)

**INTERNATIONAL SEARCH REPORT**

| International application No. |
| --- |
| **PCT/CN2022/139965** |

**C.** **DOCUMENTS CONSIDERED TO BE RELEVANT**

| Category* | Citation of document, with indication, where appropriate, of the relevant passages | Relevant to claim No. |
| --- | --- | --- |
| A | 余树民 等 (YU, Shumin et al.). "猕猴外周血液中多潜能调控因子Oct4、Nanog和Sox2基因的表达 (Expression of the Pluripotency Regulators Oct4, Nanog and Sox2 in Peripheral Blood of Rhesus Monkey)" <br> 中国兽医学报 *(Chinese Journal of Veterinary Science),* <br> Vol. 33, No. 3, 31 March 2013 (2013-03-31), <br>　　pages 389-393, see abstract, page 390, right-hand column, paragraph 1, and table 1 | 1-11 |
| A | Riccardo Monterubbianesi et al. "A comparative in vitro study of the osteogenic and adipogenic potential of human dental pulp stem cells, gingival fibroblasts and foreskin fibroblasts" <br> *Scientific Reports,* Vol. 9, 11 February 2019 (2019-02-11), <br>　　pages 1-13, see abstract | 1-11 |

Form PCT/ISA/210 (second sheet) (July 2022)

INTERNATIONAL SEARCH REPORT
Information on patent family members

International application No.

**PCT/CN2022/139965**

| Patent document cited in search report | | | Publication date (day/month/year) | Patent family member(s) | | | Publication date (day/month/year) |
|---|---|---|---|---|---|---|---|
| US | 2012288936 | A1 | 15 November 2012 | US | 2017029783 | A1 | 02 February 2017 |
| | | | | US | 10563176 | B2 | 18 February 2020 |
| | | | | US | 2020263140 | A1 | 20 August 2020 |
| | | | | US | 9453205 | B2 | 27 September 2016 |
| US | 2017313978 | A1 | 02 November 2017 | US | 2020063096 | A1 | 27 February 2020 |
| | | | | US | 11377636 | B2 | 05 July 2022 |
| | | | | WO | 2016055519 | A1 | 14 April 2016 |
| | | | | EP | 3204490 | A1 | 16 August 2017 |
| WO | 2007079533 | A1 | 19 July 2007 | None | | | |
| CN | 107988329 | A | 04 May 2018 | None | | | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202111581332X
- CN 110573607 A
- CN 110268056 A

**Non-patent literature cited in the description**

- **MONTERUBBIANESI et al.** A comparative in vitro study of the osteogenic and adipogenic potential of human dental pulp stem cells, gingival fibroblasts and foreskin fibroblasts. *Sci Rep,* 2019, vol. 9, 1761
- **SATO Y ; BANDO H ; DI PIAZZA M et al.** Tumorigenicity assessment of cell therapy products: The need for global consensus and points to consider. *Cytotherapy,* 2019, vol. 21 (11), 1095-111
- **MONTERUBBIANESI R ; BENCUN M ; PAGELLA P ; WOLOSZYK A ; ORSINI G ; MITSIADIS TA.** A comparative in vitro study of the osteogenic and adipogenic potential of human dental pulp stem cells, gingival fibroblasts and foreskin fibroblasts. *Sci Rep,* 2019, vol. 9 (1), 1761
- **HAMBILIKI F ; STROM S ; ZHANG P ; STAVREUS-EVERS A.** Co-localization of NANOG and OCT4 in human pre-implantation embryos and in human embryonic stem cells. *J Assist Reprod Genet,* 2012, vol. 29 (10), 1021-8